# EUROPÄISCHE PATENTSCHRIFT

(11) **EP 0 569 376 B1**
(45) Veröffentlichungstag und Bekanntmachung des Hinweises auf die Patenterteilung: **21.02.1996**
(21) Anmeldenummer: 92901395.1
(22) Anmeldetag: 17.12.1991
(51) Int. Cl.: C11D 1/83, C11D 10/04, A61K 7/50, A61K 7/08, C11D 1/66

(54) **FLÜSSIGWASCHMITTEL**
LIQUID WASHING AGENTS
PRODUITS LIQUIDES DE LAVAGE

(30) Priorität: 29.01.1991 DE 4102502
(43) Veröffentlichungstag der Anmeldung: 18.11.1993
(73) Patentinhaber: Henkel Kommanditgesellschaft auf Aktien, D-40191 Düsseldorf (DE)
(72) Erfinder: MEINE, Georg, D-4020 Mettmann (DE); PUCHTA, Rolf, D-5657 Haan (DE); HOFFMEISTER, Jürgen, D-40595 Düsseldorf (DE)
(86) Internationale Anmeldenummer: EP9102428
(87) Internationale Veröffentlichungsnummer: WO9213055

(56) Entgegenhaltungen:
- EP-A- 0 388 810
- EP-A- 0 403 948
- WO-A-92/03527

## Beschreibung

Die vorliegende Erfindung betrifft ein lagerstabiles dickflüssiges Flüssigwaschmittel.

Flüssige Waschmittel erfreuen sich in der letzten Zeit beim Verbraucher zunehmender Beliebtheit, da sie einige Handhabungsvorteile gegenüber pulverförmigen Waschmitteln besitzen und fettige bzw. ölige Verschmutzungen besser zu entfernen imstande sind. Der zuletzt genannte Vorteil beruht auf der Tatsache, daß flüssige Waschmittel größere Mengen der gegenüber fettigen oder öligen Verschmutzungen besonders wirksamen nichtionischen Tenside enthalten können. Der Verbraucher erwartet von derartigen Waschmitteln, daß sie in hochkonzentrierter Form angeboten werden, so daß die Waschmittel in geringerer Menge im Vergleich zu pulverförmigen Waschmitteln dosiert werden können und daß sie eine gewisse Konsistenz haben. Diese Konsistenz wird mit einem hohen Wirkstoffgehalt gleichgesetzt. Dies gilt nicht nur für Textilwaschmittel, sondern auch für Shampoos, Handwaschmittel, Geschirrspülmittel oder kosmetische Zubereitungen. Der Begriff "Flüssigwaschmittel" wird daher im folgenden für die genannten Erzeugnisse, insbesondere aber für Waschmittel für Feinwaschtextilien und für Shampoos verwendet.

Um eine Lagerstabilität auch über längere Zeit zu erreichen, enthalten Flüssigwaschmittel meist Konservierungsstoffe, die ihrerseits aber mitunter unerwünschte Nebenwirkungen haben. Neben einer guten Waschwirkung erwartet man von den genannten Flüssigwaschmitteln auch eine ausgeprägte Schaumbildung, wobei es andererseits aber wichtig ist, daß der Verbraucher den Schaum auch leicht ausspülen kann.

Es hat daher nicht an Versuchen gefehlt, Flüssigwaschmittel durch immer neue Tensidkombinationen und Schaumregulierungsmittel sowie Konservierungsmittel bereitzustellen, die hinsichtlich ihrer Waschwirkung, ihres Schaumverhaltens, ihrer Lagerstabilität und ihrer Handhabung den Erwartungen des Verbrauchers entsprechen. Es ist daher auch die Aufgabe der vorliegenden Erfindung, ein Flüssigwaschmittel mit guter Waschwirkung und guter Lagerstabilität bereitzustellen, wobei die Zusammensetzung des Waschmittels so zu wählen ist, daß die geschilderten Nachteile der Produkte nach dem Stande der Technik vermieden werden.

Aus der EP-A-70 074 sind Tensid-Kombinationen bekannt, die Alkylpolysaccharide, anionische Tenside vom Sulfat-, Sulfonat- und/oder Carboxylat-Typ in bestimmten Mischungsverhältnissen umfassen. Diese Tensidkombinationen bilden in wäßriger Lösung einen stabilen Schaum, der vollständig ausspülbar ist.

Es ist bekannt, daß man die Viskosität von Flüssigwaschmitteln häufig durch Zusatz von Elektrolyt erhöhen kann. Der Elekrolyt selbst trägt aber zum Waschergebnis nicht bei und belastet das Abwasser. Außerdem reagiert das Tensidsystem meist sehr empfindlich auf geringe Schwankungen des Elektrolytgehalts, so daß bei einem Wechsel der Rohstoffe, die ihrerseits meist unterschiedliche Mengen an Elektrolyt enthalten, die Menge an zuzusetzenden Elektrolyt stets von neuem ermittelt werden muß. Es hat daher nicht an Versuchen gefehlt, die Viskosität von Flüssigwaschmitteln durch eine geeignete Kombination an waschwirksamen Waschmittelbestandteilen einzustellen. Setzt man derartigen Flüssigwaschmitteln zur Verbesserung der Lagerstabilität niedere Alkohole zu, erhält man meist Produkte mit einer sehr geringen Viskosität. So ist Beispielsweise aus der DE-A-39 20 480 ein Flüssigwaschmittel bekannt, das Fettalkoholsulfat in Kombination mit Alkylpolyglycosid und einem hohen Anteil eines Seifengemischs enthält, das aus Salzen ungesättigter und aus Salzen gesättigter Fettsäuren in einem bestimmten Gewichtsverhältnis zueinander steht, enthält. Ein Zusatz von niederen Alkoholen zur Verbesserung der Lagerstabilität der dort beschriebenen Mittel führt zu Produkten mit einer geringen Viskosität.

Die Aufgabe der vorliegenden Erfindung besteht daher darin, lagerstabile, dickflüssige Flüssigwaschmittel mit guter Waschwirkung und ausgeprägter Schaumbildung, wobei sich der Schaum leicht ausspülen lassen muß, bereitzustellen.

Gegenstand der vorliegenden Erfindung ist daher ein Flüssigwaschmittel enthaltend anionisches Tensid, Alkylpolyglycosid und Seife, das dadurch gekennzeichnet ist, daß es
- 2 - 10 Gew.-%: Fettalkoholsulfat
- 5 - 25 Gew.-%: Alkylpolyglykosid,
- 0,1 - 9 Gew.-%: eines Kaliumsalzes von Fettsäuren mit 10 bis 20 Kohlenstoffatome und
- 3 - 8 Gew.-%: niederen Alkohol
enthält und bei Raumtemperatur eine Viskosität von 400 bis 3000 mPa·s hat.

Als Fettalkoholsulfat eignen sich die Schwefelsäuremonoester der C₁₀- bis C₂₀-Fettalkohole, insbesondere der C₁₂ - C₁₈-Fettalkohole, wie Lauryl-, Myristyl- oder Cetylalkohol, ebenso wie die Schwefelsäuremonoester der aus Kokosöl, Palm- und Palmkernöl sowie Talg gewonnenen Fettalkoholgemische, die zusätzlich noch Anteile an ungesättigten Alkoholen, z.B. Oleylalkohol, enthalten können. Eine bevorzugte Verwendung finden dabei Gemische, in denen die Anteile der Alkylreste zu 50 - 70 Gew.-% auf C₁₂, zu 18 - 30 Gew.-% auf C₁₄, zu 5 - 15 Gew.-% auf C₁₆, unter 3 Gew.-% auf C₁₀ und unter 10 Gew.-% auf C₁₈ verteilt sind.

Als Alkylpolyglykosid eignen sich Verbindungen mit einem Polymerisationsgrad (D.P.) von 1,2 bis 1,4. Der Alkylrest hat 8 - 22 Kohlenstoffatomen, vorzugsweise 12 - 18 Kohlenstoffatome. Er leitet sich von Lauryl-, Myristyl-, Cetyl- und Stearylalkohol sowie von technischen Fraktionen, die vorzugsweise gesättigte Alkohole enthalten, ab. Besonders bevorzugt ist der Einsatz von Alkylglucosiden, deren Alkylrest zu 50 - 70 Gew.-% C₁₂ und 18 - 30 Gew.-% C₁₄ enthält. Derartige Alkylglykoside sind handelsübliche Substanzen, die durch sauer katalysierte Umsetzung von Glykosiden und Fettalkohol hergestellt werden.

Weiterhin ist ein Kaliumsalz von Fettsäuren mit 10 bis 20 Kohlenstoffatomen enthalten. Eine geeignete Fettsäure ist beispielsweise das Kaliumsalz der Kokosfettsäure. Kaliumsalze von Fettsäuren mit 10 bis 20 Kohlenstoffatomen werden im weiteren als "Seife" bezeichnet.

Der in den erfindungsgemäßen Flüssigwaschmitteln zu verwendende Alkohol ist vorzugsweise ein Alkanol oder ein Alkandiol mit 1 bis 3 Kohlenstoffatomen. Bevorzugt ist von diesen Alkoholen Ethanol. Ein Zusatz von Ethanol zu den erfindungsgemäßen Mitteln bewirkt eine ausgezeichnete Lagerstabilität auch über einen längeren Zeitraum hinweg.

Feinwaschmittel für Textilien enthalten vorzugsweise 3 - 8 Gew.-% der oben genannten Seife. Shampoos hingegen benötigen eine geringere Menge an Seife. Besonders ausgewogene Eigenschaften hat ein Shampoo, daß 0,1 - 1,0 Gew.-% Seife enthält.

Bei der Herstellung der genannten Flüssigwaschmittel kann man entweder die Seife als solche einsetzen, oder man kann die entsprechende Fettsäure und Kalilauge separat zusetzen. Das zuletzt genannte Verfahren hat den Vorteil, daß man damit innerhalb gewisser Grenzen den pH-Wert des Flüssigwaschmittels regulieren kann. Mitunter kann es zweckmäßig sein, daß die erfindungsgemäßen Flüssigwaschmittel noch zusätzlich weitere Tenside enthalten. Als weitere Tenside sind vor allen Dingen Fettalkoholethoxylate sowie amphotere Tenside und Tenside vom Betain-Typ zu nennen. Derartige Tenside können bis zu 8 Gew.-% in den erfindungsgemäßen Flüssigwaschmitteln enthalten sein. Darüberhinaus können außerdem noch weitere übliche Bestandteile von Flüssigwaschmitteln, beispielsweise Perlglanzmittel, Farbstoffe, Duftstoffe oder Enzyme sowie Gerüststoffe enthalten sein. Flüssigwaschmittel nach der vorliegenden Erfindung haben eine Viskosität von 400 bis 3000 mPa·s bei Raumtemperatur. Es ist in hohem Maße überraschend, daß es möglich ist, eine derartige Viskosität ohne Zusatz von Verdickungsmitteln und mit einem verhältnismäßig geringen Anteil an Seife in Gegenwart der die Lagerstabilität verbessernden niederen Alkohole einzustellen.

### Beispiele

### Beispiel 1

Es wurde ein flüssiges Waschmittel der folgenden Zusammensetzung hergestellt:
- 5,0 Gew.-%: C₁₂-C₁₈-Fettalkoholsulfat-Na
- 10,0 Gew.-%: C₁₂-C₁₄-Alkylpolyglucosid-1,4
- 5,0 Gew.-%: C₁₂-C₁₈-Kokosölseife-K
- 5,0 Gew.-%: Ethanol
- 1,0 Gew.-%: Duftstoff
- Rest: Wasser

Dabei wurden die aufgeschmolzene Seife und Fettalkoholsulfat in auf 80°C erwärmtes Wasser eingerührt und nach Abkühlen auf 50°C Ethanol und Alkylpolyglucosid untergemischt. Der Duftstoff wurde nach Erkalten zugeführt.

Dieses Produkt hatte einen pH-Wert von 8,0 und eine Viskosität von 840 mPa·s bei Raumtemperatur. Die Lagerstabilität war ausgezeichnet; zusätzliche Konservierungsmittel waren nicht erforderlich.

Mit diesem Waschmittel wurde normal verschmutzte Haushaltswäsche in einer automatischen Trommelwaschmaschine (Typ AEG 570) im 1-Laugen-programm für pflegeleichte Wäsche gewaschen. Die Wasserhärte betrug 16°d, pro Maschinenfüllung wurden 120 g Waschmittel eingesetzt. Die Waschversuche wurden bei 30, 40 und 60°C durchgeführt und die Waschwirkung durch visuelle Beurteilung und durch Ermittlung des Reflexionswertes der gewaschenen Textilien ermittelt. Als Vergleich diente ein hochwertiges handelsübliches flüssiges Feinwaschmittel, das unter gleichen Bedingungen eingesetzt wurde. Außer der Waschwirkung wurde die Schaumhöhe und das Ausspülverhalten des Schaums nach 3 Spülgängen bewertet. Dabei wurden keine signifikanten Unterschiede in den Leistungsmerkmalen beider Waschmittel festgestellt.

Durch Verringerung des Seifenanteils läßt sich die Viskosität überraschenderweise erhöhen, falls dies wünschenswert sein sollte.

### Beispiel 2

Die Steuerbarkeit des Schäumverhaltens durch die Seifenkonzentration in den erfindungsgemäßen Rezepturen läßt sich ausnutzen, um beispielsweise höherviskose Shampoos zu formulieren.

Es wurde ein lagerstabiles Shampoo der folgenden Zusammensetzung hergestellt
- 1,0 Gew.-%: Ethylenglykolstearat
- 5,0 Gew.-%: Fettalkoholsulfat
- 10,0 Gew.-%: Alkylpolyglucosid
- 0,5 Gew.-%: Seife
- 5,0 Gew.-%: Ethanol
- 1,0 Gew.-%: Duftstoff
- Rest: Wasser

Dieses Shampoo hatte eine Viskosität von 1435 mPa·s und einen pH-Wert von 7,9. Das Schäum- und Ausspülverhalten war ausgezeichnet und entsprach dem eines hochwertigen handelsüblichen Shampoos.

## Patentansprüche

1. Flüssigwaschmittel enthaltend anionisches Tensid, Alkylpolyglykosid und Seife, dadurch gekennzeichnet, daß es
2 - 10 Gew.-% Fettalkoholsulfat
5 - 25 Gew.-% Alkylpolyglykosid,
0,1 - 9 Gew.-% eines Kaliumsalzes von Fettsäuren mit 10 bis 20 Kohlenstoffatomen und
3 - 8 Gew.-% niederen Alkohol
enthält und bei Raumtemperatur eine Viskosität von 400 bis 3000 mPa·s hat.

2. Flüssigwaschmittel nach Anspruch 1, dadurch gekennzeichnet, daß der niedere Alkohol ein Alkanol oder Alkandiol mit 1 bis 3 Kohlenstoffatomen, vorzugsweise Ethanol ist.

3. Flüssigwaschmittel nach einem der Ansprüche 1 oder 2, dadurch gekennzeichnet, daß es 3 bis 8 Gew.-% der Kaliumseife enthält.

4. Flüssigwaschmittel nach einem der Ansprüche 1 bis 3, dadurch gekennzeichnet, daß es 0,1 bis 1 Gew.-% der Kaliumseife enthält.

5. Flüssigwaschmittel nach einem der Ansprüche 1 bis 4, dadurch gekennzeichnet, daß es zusätzlich bis zu 8 Gew.-% weitere Tenside enthält.

6. Flüssigwaschmittel nach einem der Ansprüche 1 bis 5, dadurch gekennzeichnet, daß es weitere übliche Bestandteile von Flüssigwaschmitteln enthält.

## Claims

1. A liquid detergent containing anionic surfactant, alkyl polyglycoside and soap, characterized in that it contains
2 to 10% by weight of fatty alcohol sulfate
5 to 25% by weight of alkyl polyglycoside
0.1 to 9% by weight of a potassium salt of C₁₀₋₂₀ fatty acids and
3 to 8% by weight of lower alcohol
and has a viscosity of 400 to 3000 mPa·s at room temperature.

2. A liquid detergent as claimed in claim 1, characterized in that the lower alcohol is an alkanol or alkanediol containing 1 to 3 carbon atoms, preferably ethanol.

3. A liquid detergent as claimed in claim 1 or 2, characterized in that it contains 3 to 8% by weight of the potassium soap.

4. A liquid detergent as claimed in any of claims 1 to 3, characterized in that it contains 0.1 to 1% by weight of the potassium soap.

5. A liquid detergent as claimed in any of claims 1 to 4, characterized in that it additionally contains up to 8% by weight of other surfactants.

6. A liquid detergent as claimed in any of claims 1 to 5, characterized in that it contains other typical ingredients of liquid detergents.

## Revendications

1. Produit de lavage liquide contenant un tensioactif anionique, un alkylpolyglycoside et un savon, qui est caractérisé en ce qu'il contient:
2 à 10 % en poids de sulfate d'alcool gras,
5 à 25 % en poids d'alkylpolyglycoside,
0,1 à 9 % en poids d'un sel de potassium d'acides gras comportant 10 à 20 atomes de carbone et
3 à 8 % en poids d'alcool inférieur
et possède à température ambiante une viscosité de 400 à 3000 mPa.s.

2. Produit de lavage liquide selon la revendication 1, caractérisé en ce que l'alcool inférieur est un alcanol ou un alcanediol comportant 1 à 3 atomes de carbone, de préférence l'éthanol.

3. Produit de lavage liquide selon la revendication 1 ou 2, caractérisé en ce qu'il contient 3 à 8 % en poids de savon de potassium.

4. Produit de lavage liquide selon une des revendications 1 à 3, caractérisé en ce qu'il contient 0,1 à 1 % en poids de savon de potassium.

5. Produit de lavage liquide selon une des revendications 1 à 4, caractérisé en ce qu'il contient en plus jusqu'à 8 % en poids d'autres tensioactifs.

6. Produit de lavage liquide selon une des revendications 1 à 5, caractérisé en ce qu'il contient d'autres constituants usuels de produits de lavage liquides.
